Europäisches Patentamt

European Patent Office

Office européen des brevets

(19)

(11) Publication number: **0 409 488 A2**

# EUROPEAN PATENT APPLICATION

(21) Application number: 90307629.7

(22) Date of filing: 12.07.90

(51) Int. Cl.5: **C08B 37/06**, C12P 19/04

(30) Priority: 17.07.89 JP 184311/89

(43) Date of publication of application:
23.01.91 Bulletin 91/04

(84) Designated Contracting States:
AT BE CH DE DK ES FR GB GR IT LI LU NL SE

(71) Applicant: TAKEDA CHEMICAL INDUSTRIES, LTD.
3-6, Doshomachi 2-chome Chuo-ku
Osaka 541(JP)

(72) Inventor: Haze, Akira
4-45, Seiwadainishi 5-chome
Kawanishi, Hyogo 666-01(JP)
Inventor: Hashimoto, Koichi
22-25, Kamo 2-chome
Takaishi, Osaka 592(JP)
Inventor: Miyanagi, Kazuki
1025, Saikujo, Shikata-cho
Kakogawa, Hyogo 675(JP)
Inventor: Kanegae, Yukihiro
15-18, Nakano 1-chome, Nishi-ku
Kobe, Hyogo 673(JP)

(74) Representative: Lewin, John Harvey et al
ELKINGTON AND FIFE Beacon House 113
Kingsway
London WC2B 6PP(GB)

(54) Method for producing a heat-gelable beta-1,3-glucan.

(57) A heat-nongelable $\beta$-1,3-glucan is made into a heat-gelable $\beta$-1,3-glucan by a method which comprises dissolving the heat-nongelable $\beta$-1,3-glucan with alkali and adjusting the solution to a pH not exceeding pH 10 to cause precipitation of the heat-gelable $\beta$-1,3-glucan.

EP 0 409 488 A2

# METHOD FOR PRODUCING A HEAT-GELABLE $\beta$-1,3-GLUCAN

## FIELD OF THE INVENTION

This invention relates to a method for producing a heat-gelable $\beta$-1,3-glucan which is of use in the field of food and chemical industries.

## BACKGROUND OF THE INVENTION

In the natural kingdom, there exist many cells containing heat-nongelable $\beta$-1,3-glucan granules. For example, there is known a method for recovery of such glucan granules from Euglena , a genus of protozoa (Japanese Patent Laid Open Publication No. 37297/1989). These glucan granules are high in crystallinity and characteristically do not swell in hot water (Carbohydrate Research, 75 (1979) 231-242). On the other hand, as a $\beta$-1,3-glucan which is extracellularly excreted by microorganisms of, for example, the genus Alcaligenes or Agrobacterium , there is known curdlan, which gels when heated in hot water (New Food Industry, 20 , 49, 1978).

As is described above, there are two kinds of $\beta$-1,3-glucan, i.e. heat-nongelable $\beta$-1,3-glucan and heat-gelable $\beta$-1,3-glucan.

The heat-nongelable $\beta$-1,3-glucan cannot serve the same purposes as curdlan, for example in the aspects of gelation, water holding and binding properties. Thus, there is no method for producing a heat-gelable $\beta$-1,3-glucan advantageously on a commercial scale from the heat-nongelable $\beta$-1,3-glucan granules obtained from the cells.

## OBJECT OF THE INVENTION

A object of the present invention is to provide a method for producing a heat-gelable $\beta$-1,3-glucan from a heat-nongelable $\beta$-1,3-glucan advantageously on a commercial scale.

## SUMMARY OF THE INVENTION

The present invention relates to a method for producing a heat-gelable $\beta$-1,3-glucan which comprises dissolving a heat-nongelable $\beta$-1,3-glucan with alkali and adjusting the solution to a pH not exceeding pH 10 to cause precipitation of the heat-gelable $\beta$-1,3-glucan.

## DETAILED DESCRIPTION OF THE INVENTION

The heat-nongelable $\beta$-1,3-glucan granules to be employed in this invention are obtained by the conventional method from the cells of animals, plants, microorganisms, etc. which contain such glucan granules. The cells are preferably those obtained by culture of protozoa, such as Euglena, and examples of protozoa of this genus Euglena include Euglena gracilis and Euglena gracilis var. bacillars .

To be specific, there may be mentioned such strains as Euclena gracilis Klebs NIES-47, Euglena gracilis Klebs NIES-48, Euglena gracilis var. bacillaris Pringsheim NIES-49 and so on. These strains are known protozoans deposited with Global Environmental Forum at the National Institute of Pollution Research.

These Euglena protozoa are ready to undergo mutation upon irradiation with X-rays, ultraviolet rays or other radiation, or treatment with a mutagenic agent. Such mutants can also be employed in the method of this invention insofar as they have the ability to accumulate the heat-nongelable $\beta$-1,3-glucan in their cells.

With regard to culture media for Euglena protozoans, there may be mentioned Koren-Hutner medium-

(Koren, L.E.C., Hutner, S.H., Journal of Protozoology 14 , Supplement 17, 1967), the medium of Kuragano et al.(Japanese Patent Laid Open Publication No. 58064/1985) and the medium of Kitaoka et al. (Japanese Patent Laid Open Publication No. 37297/1989). By growing Euglena in such a medium or a suitable version of the medium and harvesting the cells of Euglena, there can be obtained cells containing the heat-nongelable $\beta$-1,3-glucan.

The cells containing such $\beta$-1,3-glucan are wet-disrupted to liberate heat-nongelable $\beta$-1,3-glucan granules. This disruption can be advantageously carried out using a pressure cell disintegrator at a pressure of 300 to 600 kg/cm$^2$ or by means of an ultrasonic disintegrator. The disrupted cells are then treated with alkali to dissolve heat-nongelable $\beta$-1,3-glucan granules. For this treatment, the heat-nongelable $\beta$-1,3-glucan granules may be separated from the disrupted cell fraction beforehand or the system containing both the glucan granules and disrupted cells may be used as it is. In the latter case, the disrupted cells, which are insoluble, can be removed with a commercially useful efficiency by applying the foaming procedure described hereinafter concurrently with the alkali dissolution procedure. While there is virtually no limitation on the kind of alkali that can be employed, lithium hydroxide, sodium hydroxide, potassium hydroxide or ammonium hydroxide is generally preferred. The concentration of alkali required for dissolution of the heat-nongelable $\beta$-1,3-glucan granules need not be much higher than 1N but when the system contains both the heat-nongelable $\beta$-1,3-glucan granules and cell fragments, a concentration of about 3-5N is preferred, for it assists in the production of a foam. The preferred concentration of the heat-nongelable $\beta$-1,3-glucan granules for the dissolution procedure is about 0.1 to 1 weight percent.

After dissolution of the heat-nongelable $\beta$-1,3-glucan granules, the alkali-insoluble fraction is removed. This insoluble fraction is predominantly composed of fragments of Euglena or other cells. This removal of insoluble matter can be accomplished with high efficiency and commercial advantage by bubbling air into the alkalinized system to cause production of a foam and thereby let the cell fragments be entrained by the foam. This procedure is described in further detail below.

Using a turbine mixer or the like or by means of a continuous foaming machine, air is forced or bubbled into the alkalinized slurry containing disrupted cells to produce a foam. The volume of the foam is generally not less than 30% of the volume of the slurry and preferably in the range of 50 to 70%. While the foam is rather readily produced at the above-mentioned alkali concentration, egg albumin (0.1 to 0.5% based on slurry volume) or a surfactant (e.g. a sugar ester) may be added to the system for the purpose of stabilizing the foam for more discrete removal of the foam.

The foam separation is preferably carried out at ambient temperature (20-30°C) and the time required for the formation of a discrete interface, that is to say the foam layer formation time, is not longer than 3-4 hours.

For separation of the foam, a batch separation equipment on a tank scale or, for a system with a more or less unstable foam layer, a continuous separation equipment comprising an elongated vessel can be employed.

A heat-nongelable $\beta$-1,3-glucan solution free of insolubles can thus be obtained. This solution is then adjusted to pH 10 or less to cause $\beta$-1,3-glucan to separate out. In consideration of uses for the precipitated $\beta$-1,3-glucan, this precipitation procedure is preferably carried out in the neighborhood of neutral pH and particularly at pH 6-6.5. While an appropriate pH modifying agent can be selectively employed, it is generally advantageous to use a mineral acid such as hydrochloric acid, sulfuric acid, nitric acid and phosphoric acid. While the precipitated $\beta$-1,3-glucan is heat-gelable, it is more advantageous, for commercial purposes, to further subject it to desalting, concentration and drying to provide a powdery preparation. By way of illustration, the above solution containing the precipitated glucan is concentrated by centrifugation and washed with water to give a desalted concentrate (2-4%). This concentrate can be either spray-dried to give a dry powder or frozen, thawed in a hydrophilic organic solvent, dehydrated and dried by heating in vacuo to give a dry powder.

The $\beta$-1,3-glucan produced in accordance with the present invention is heat-gelable unlike paramylon and other known heat-nongelable $\beta$-1,3-glucan powders, and by exploiting this characteristic, can be advantageously utilized in food and chemical industries. For example, it can be used in the same applications as curdlan.

Examples

The following examples are further illustrative of the present invention.

Example 1

A 5-liter jar fermenter was charged with 3 liters of Koren-Hutner medium, which was then sterilized at 121°C for 20 minutes. The fermenter was inoculated with 150 ml of a seed culture of Euglena gracilis Klebs NIES-48 in the same medium. The incubation was carried out in the dark with a rotational speed of 400 rpm, with an aeration of 1 ℓ/min at 28°C for 96 hours. Two jars yielded 6.15 liters of culture broth. The yield of cells amounted to 2% on a dry basis and this dry product contained 20% of β-1,3-glucan.

(1) Using a desk-top centrifuge (Model RS-18GL, Tomy Precision Industries, Ltd., Japan), 5.0 liters of the culture broth was centrifuged at 6,000 rpm to give 1 kg of a concentrated Euglena paste.

(2) To this wet-cell paste was added a sufficient amount of water to make 2 liters and the mixture was homogenized using a high-pressure homogenizer (trade name: Manton-Gaulin, APV Gaulin, USA) at a pressure of 300 atmospheres, whereby the Euglena cells were disrupted to liberate glucan granules.

(3) To the resulting slurry of disrupted cells was added 30% sodium hydroxide to a concentration of 3N and the mixture was heated at 60°C, whereby the β-1,3-glucan granules were dissolved in 4 hours.

(4) This alkalinized solution of disrupted cells (3.5 ℓ) was cooled to about 30°C. Then, 0.2% by volume of a non-heat-gelable egg albumin (manufactured by Takeda Chemical Industries, Ltd., Japan) was added and the mixture was agitated in a turbine stirrer (5 ℓ) to produce a foam. The agitation was discontinued when the volume of the foam had reached about 50% of the charge and the system was allowed to stand for 3-4 hours. In this manner, fragments of protozoan cells and other insolubles were adsorbed on the foam and, as such, can be removed as scrums. The resulting β-1,3-glucan solution (about 3.0 ℓ) freed of disrupted cell fragments was withdrawn from the bottom of the stirrer vessel.

(5) This β-1,3-glucan solution was adjusted to pH 6-6.5 with 4N-hydrochloric acid to thereby precipitate the β-1,3-glucan.

(6) This solution containing the precipitated glucan was centrifugally concentrated with the Tomy centrifugal machine mentioned above (8,000 rpm x 10 min.) to give a neutral concentrate (solid content 2.5%). This concentrate was re-slurried with sufficient water to make 3 liters to thereby give a desalted neutral concentrate (solid content 3%). This concentrate was diluted with water again and subjected to the same procedure as above to give 300 g of a neutral concentrate (solid content 3%).

(7) The above concentrate (300 g) was frozen batchwise at -20°C and, then, thawed at room temperature with addition of twice its volume of alcohol, followed by vacuum dehydration with a glass filter to give 24 g of a dehydrated product (solid content about 25%). This dehydrated product was dried in vacuo at 60°C to give dry flakes. The dry flakes were then pulverized with a desk-top pulverizer to give 7 g of β-1,3-glucan powder.

The quality parameters of this β-1,3-glucan powder (Sample 1) were shown below alongside those of the β-1,3-glucan granules prior to alkali treatment (Sample 2), in Table 1.

Table 1

| Quality parameters of powders | | |
|---|---|---|
| | Sample 2 | Sample 1 |
| Mean particle size (μ) | 3 | 70 |
| Moisture (%) | 2 | 5 |
| Solution (optical density, 660 nm) | - | 80 |
| Residue on ignition (%) | 0.05 | 1.2 |
| Gel strength (g/cm$^2$) | | |
| (2% Concentration) | Not gelable | 160 |
| (3% Concentration) | Not gelable | 400 |
| (4% Concentration) | Not gelable | 750 |

Gel strength: To β-1,3-glucan was added 10 ml of water to the concentration indicated and the mixture was homogenized with a potter's homomixer and degassed in vacuo. The homogenate was placed in a test tube (1.6 cm dia.) and heated in a boiling water bath for 10 minutes. The tube was cooled with tap water for 30 minutes and the resulting gel was cut to a height of 10 mm. The gel strength of this specimen was

4

measured with a curd meter (Iio Type, Iio Electric Co., Japan).

It will be apparent from the data in Table 1 that whereas the $\beta$-1,3-glucan granules from a Euglena culture broth (generic name: paramylon) is not heat-gelable, the $\beta$-1,3-glucan powder produced in accordance with the present invention is sufficiently heat-gelable, giving a gel strength comparable to that of ordinary gelling agents (agar, gelatin, curdlan, etc.).

## Example 2

Using a 5-liter jar fermenter, Euglena glacilis Klebs NIES-48 was cultured in the same manner as Example 1 to give 3.1 liters of a culture broth. A 2-liter portion of this broth was centrifuged with a free-standing centrifuge (8,000 rpm x 10 min.) to give a cell paste. This cell paste was diluted with water to make 400 ml and treated with a sonicator (T-A-4280, 10kc x 10 min.) for disruption of cells.

(1) To 200 ml of this slurry was added 30% sodium hydroxide to a concentration of 3N and the $\beta$-1,3-glucan granules were dissolved at 60°C in about 5 hours. The solution was centrifuged with the Tomy centrifuge (8,000 rpm x 10 min.) to remove insolubles and the resulting solution was neutralized to pH 6.0 with 35% HCl. The neutral solution was desalted and concentrated by means of the Tomy centrifuge (8,000 rpm x 10 min.) to give about 12 g of a paste. This paste was diluted with water to make 200 ml and subjected to the same desalting and concentration procedure twice to give 12 g of a desalted concentrate. This concentrated paste was frozen at -20°C and, then, thawed with addition of twice its volume of ethanol and subjected to Nutsche vacuum dehydrative filtration to give 1.4 g of a dehydrated product. This dehydrated product was further dried in vacuo at 50°C to give a porous greenish dry product. It was then pulverized in a mortar to give 0.5 g of powder.

(2) Prior to alkali dissolution in the above procedure (1), the slurry was decolorized with 100% by volume of 99% ethanol and centrifuged with the Tomy centrifuge to give a decolorized disrupted cell paste. This paste was re-slurried with water to make 200 ml and degreased with 100% by volume of n-hexane. The aqueous layer was subjected to the same procedure as described in (1) to give 0.5 g of a porous white powder.

Each of the powders (1) and (2) was dispersed in water to make a 2% dispersion whichwas then homogenized, heated at 100°C and cooled to ambient temperature. The gel strengths of these gels were 150 g/cm² and 180 g/cm², respectively.

In the above manner, heat-gelable $\beta$-1,3-glucans can be obtained from the heat-nongelable $\beta$-1,3-glucan granules derived from Euglena.

## Example 3

In 200 ml of 1N-aqueous sodium hydroxide solution was dissolved 2 g of paramylon powder with stirring and the solution was adjusted to pH 6.0 with 4N-HCl to thereby precipitate the $\beta$-1,3-glucan. The resulting neutral slurry was concentrated with the Tomy centrifuge (8,000 rpm x 10 min.) and the concentrate was re-slurried with 200 ml of water. This slurry was concentrated by centrifuging in the same manner as above to give 50 g of a paste. This paste was frozen at -20°C and thawed with addition of 200% by volume of ethanol, followed by suction-filtration through filter paper to give 8 g of a dehydrated product. This product was further dried in vacuo at 60°C to give about 2 g of a porous dry product.

The gel strength of the resulting $\beta$-1,3-glucan powder as determined by the above-described method was 200 g/cm² for 2% concentration. Whereas this glucan powder is thus heat-gelable, the starting material paramylon has no gelation property.

## Claims

1. A method for producing a heat-gelable $\beta$-1,3-glucan which comprises dissolving a heat-nongelable $\beta$-1,3-glucan with alkali and adjusting the solution to a pH not exceeding pH 10 to cause precipitation of the heat-gelable $\beta$-1,3-glucan.

2. A method according to claim 1, wherein the solution is prepared by treating a wet disruption product of cells containing the heat-nongelable $\beta$-1,3-glucan with alkali to dissolve the glucan granules and then removing insoluble matter.

3. A method according to claim 1, wherein alkali is alkali metal hydroxide or ammonium hydroxide.

4. A method according to claim 2, wherein the cell is a cell of protozoa.

5. A method according to claim 2, wherein the insoluble matter is removed by foaming.

6. A method according to claim 3, wherein the alkali metal hydroxide is sodium hydroxide or potassium hydroxide, lithium hydroxide.

7. A method according to claim 4, wherein the protozoa is Euglena.

8. A method according to claim 7, wherein the Euglena is Euglena gracilis Klebs NIES-48.